# EUROPEAN PATENT APPLICATION

(11) **EP 2 574 320 A1**
(43) Date of publication of application: **03.04.2013**
(21) Application number: 12181382.8
(22) Date of filing: 22.08.2012
(51) Int. Cl.: A61F 13/10, A61F 13/08, A61H 9/00

(54) **Compression Sleeve**

(30) Priority: 30.09.2011 US 201113249465
(71) Applicant: Covidien LP, Mansfield, MA 02048 (US)
(72) Inventor: Deshpande, Manish, Massachusetts 02021 (US); Bruehwiler, Michel, Massachusetts 02461 (US); Rosen, Melissa, Massachusetts 01970 (US)
(74) Representative: Gray, James

(57) **Abstract**

A compression sleeve configured for a conforming fit to a portion of a wearer's body, such as a leg. The compression sleeve includes at least one inflatable bladder. The sleeve has a periphery including at least six edges and is configured to wrap around the wearer's leg so that the bladder encircles the limb for applying compression therapy to the limb. The shape of the sleeve conforms to the limb of the wearer and keeps the sleeve in place on the limb.

## Description

### FIELD OF THE INVENTION

The present application claims the benefit of and priority to US Patent Application No. 13/249,465 filed September 30, 2011, the entire contents of which is incorporated by reference herein.

### BACKGROUND OF THE INVENTION

The present invention generally relates to compression sleeves, and in particular to a compression garment.

Active compression garments for applying intermittent compression to a limb have many applications, including deep vein thrombosis prophylaxis, edema prevention, and aiding in wound healing. The performance of such compression garments is sensitive to the ability of the sleeve to retain its initial tension and the ability of the inflatable bladders to retain their original position around the leg. This can be very difficult when the compression garments are used during and after ambulation, such as walking, sitting, standing, and rolling over. The garments tend to slide down the limb (in particular, the leg) causing discomfort and misalignment of inflatable bladders within the garment with respect to the leg. Thus, a garment needs to "stay up" in use. The stay-up capability of such compression garments is very beneficial for effective compression treatment.

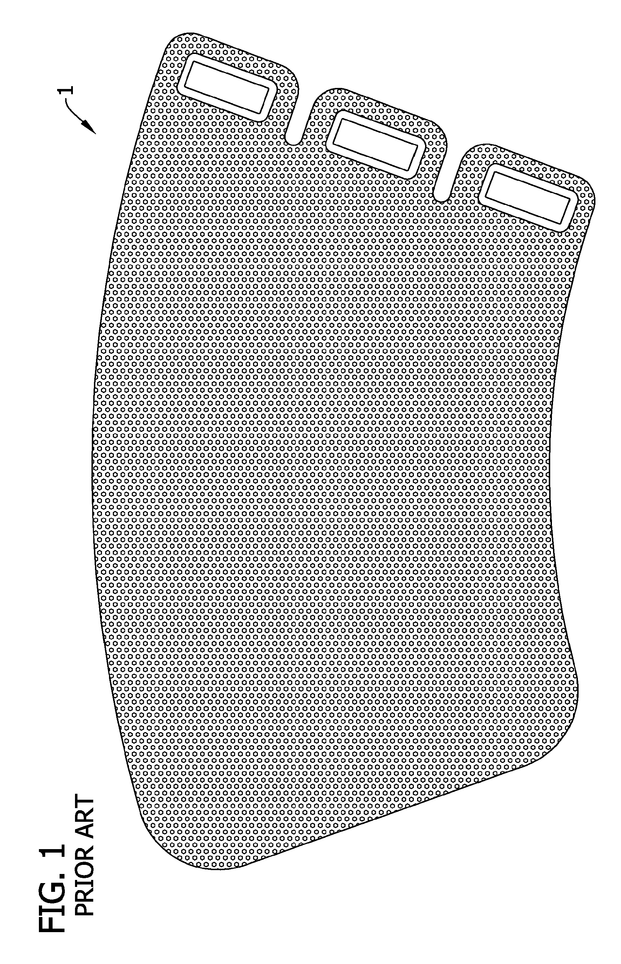

The stay-up ability of the compression garment or "sleeve" is complicated by the natural shape of the leg. A uniformly shaped garment, like many sleeves in the prior art, is naturally disposed to moving downwards when mounted on a leg. Figure 1 illustrates an example of such a uniformly shaped sleeve 1 of the prior art. There exists a need for a sleeve having greater "stay-up" capability than the sleeves in the prior art.

### SUMMARY OF THE INVENTION

In one aspect of the present invention, a compression sleeve for applying compression therapy to a wearer's body generally comprises a panel of conformable material including an upper proximal edge, a lower distal edge, first and second opposing side edges and a longitudinal axis extending from the proximal edge to the distal edge. A first upper transition edge extends from the upper proximal edge to a first side edge, the first upper transition edge having one of a generally concave or linear shape. A second lower transition edge extends from the lower distal edge to the first side edge, the second lower transition edge having a generally concave or linear shape. At least one inflatable bladder is attached to the panel, and a fastener is able to secure the panel in a wrapped configuration around the wearer's body.

In another aspect of the present invention, a compression garment for applying compression therapy to a limb of a wearer generally comprises a compression sleeve including at least one inflatable bladder. The compression sleeve has a periphery including at least six edges, and is configured to wrap around the wearer's limb so that the at least one bladder encircles the limb for applying compression therapy to the limb. The shape of the sleeve conforming to the limb of the wearer and keeping the sleeve in place on the limb.

Other objects and features will be in part apparent and in part pointed out hereinafter.

### BRIEF DESCRIPTION OF THE DRAWINGS

Fig. 1 is a rear view of a compression sleeve of the prior art.
Fig. 2 is a front view of a compression sleeve of the present invention;
Fig. 3 is a front view of the compression sleeve having portions of the sleeve broken away;
Fig. 4 is a rear view of the compression sleeve;
Fig. 5 is an enlarged fragmentary elevation illustrating loop material on an outer cover of the sleeve;
Fig. 6 is a perspective of the compression sleeve wrapped around a leg of a wearer;
Fig. 7 is a rear view of a second embodiment of a compression sleeve of the present invention;
Fig. 8 is a rear view of a third embodiment of a compression sleeve of the present invention;
Fig. 9 is a rear view of a fourth embodiment of a compression sleeve of the present invention;
Fig. 10 is a rear view of a fifth embodiment of a compression sleeve of the present invention;
Fig. 11 is a rear view of a sixth embodiment of a compression sleeve of the present invention;
Fig. 12 is a rear view of a seventh embodiment of a compression sleeve of the present invention; and
Fig. 13 is a perspective of the compression sleeve of the seventh embodiment wrapped around a leg of a wearer.

Corresponding reference characters indicate corresponding parts throughout the drawings.

### DESCRIPTION OF THE PREFERRED EMBODIMENTS

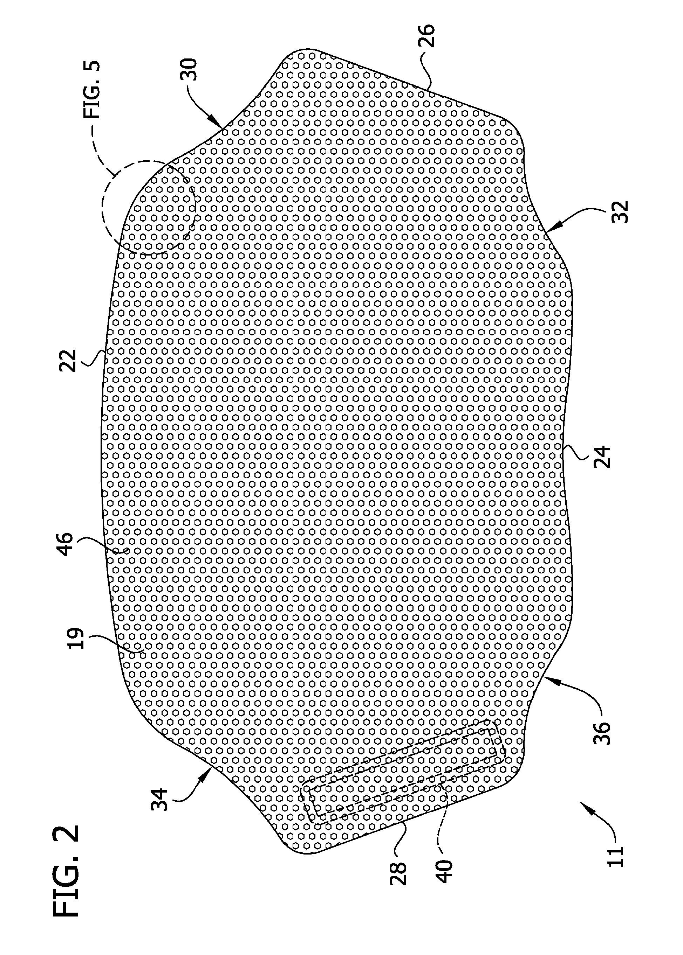

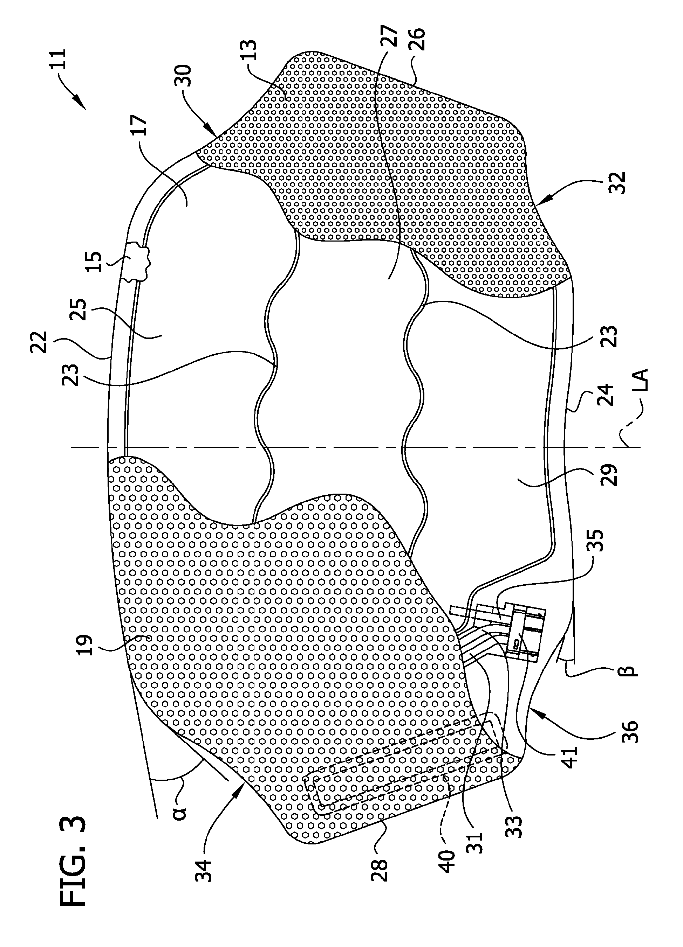

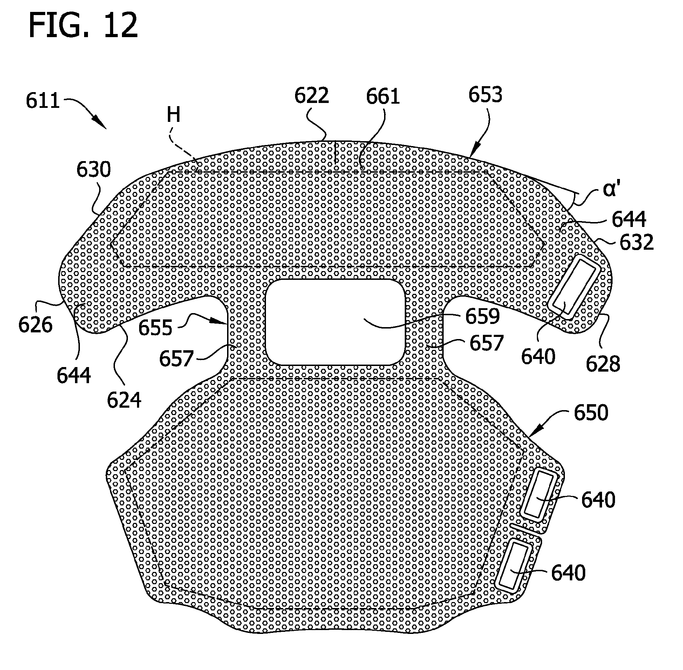

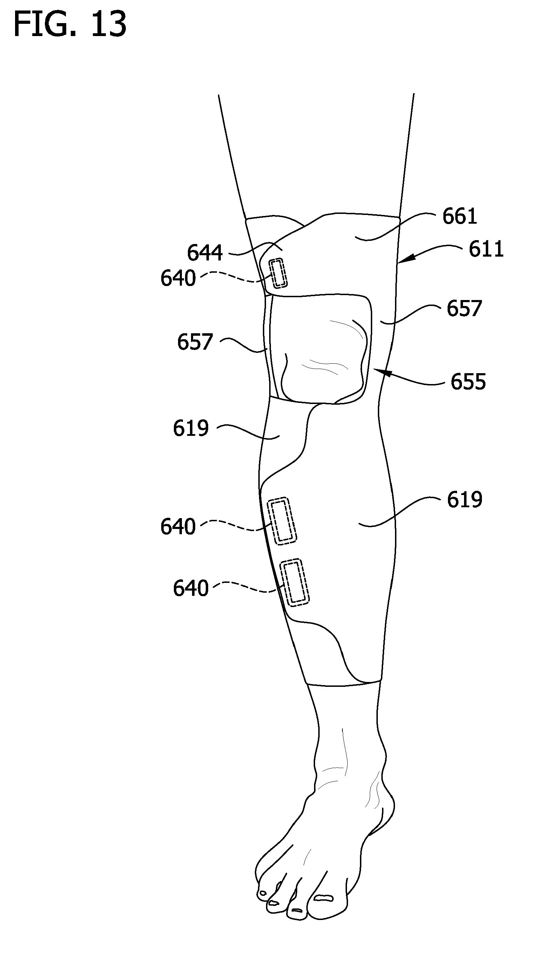

Referring now to the figures, in which like reference numerals identify identical or corresponding elements, various embodiments of the presently disclosed compression sleeve will now be described in detail. Referring in particular to Figs. 2 and 3, a compression garment (or "sleeve") of the present invention is generally indicated at 11. The compression garment is capable of being connected to a controller (not shown) that supplies compressed air to the garment for applying repeated, sequential compression therapy to a leg of a wearer. A compression sleeve may be configured to fit on other parts of the body within the scope of the present invention. The sleeve 11 is a knee-length sleeve sized and shaped for being disposed around a leg of the wearer. It will be understood that the compression sleeve may come in different configurations, such as a thigh-length sleeve. One embodiment of a thigh-length sleeve (described more fully hereinafter) is illustrated in Figs. 12 and 13. Other types of compression devices for being disposed about other limbs of the wearer's body are within the scope of this invention as well. These include devices that do not apply compression repeatedly and/or sequentially.

Referring to Fig. 3, the compression sleeve 11 comprises four layers secured together. The compression sleeve 11 comprises an inner layer, generally indicated at 13, on which a first intermediate layer (a first bladder layer), generally indicated at 15, is overlaid. A second intermediate layer (a second bladder layer), generally indicated at 17, overlies the first intermediate layer 15 and is secured thereto. An outer layer or cover generally indicated at 19 overlies the second intermediate layer 17. In the illustrated embodiment, the inner layer 13 and outer layer 19 define a panel having peripheral edges that define the periphery of the sleeve. It will be understood that the panel may have a greater or fewer number of components. The layers 13-19 may be secured together in any suitable manner as by radiofrequency welding, adhesive, or other chemical and/or mechanical process. In the illustrated embodiment, the layers 13-19 are secured about a periphery of the sleeve 11 including an upper proximal edge 22, a lower distal edge 24 and opposing side edges 26, 28. A longitudinal axis LA extends from the upper proximal edge 22 to the lower distal edge 24.

The periphery further includes first upper transition edge 30 extending from the upper proximal edge 22 to first side edge 26. A second lower transition edge 32 extends from the lower distal edge 24 to the first side edge 26. The first upper transition edge 30 extends from the upper proximal edge 22 at about a 40 degree angle α (only one angle is shown). The first upper transition edge 30 has a generally concave shape. The second lower transition edge 32 has a generally concave shape.

A third upper transition edge 34 extends from the upper proximal edge 22 to the second side edge 28. A fourth lower transition edge 36 extends from the lower distal edge 24 to the second side edge 28. The third upper transition edge 34 extends at an angle or about 40° (angle α) and the fourth lower transition edge 36 extend from the lower distal edge 24 at about a 40 degree angle β (only one angle is shown). In the illustrated embodiment, the overall shape of the upper and lower transition edges 30, 32, 34, 36 is generally concave. However, the overall shape of the upper and lower transition edges 30, 32, 34, 36 can have over configurations, such as generally linear, without departing from the scope of the invention. There are corners between the various peripheral edges of the sleeve 11. The corners each have a small radius of curvature and are generally convex in shape.

In use, the inner layer 13 is disposed most adjacent to the limb of the wearer and is in contact with the limb of the wearer, and the outer cover 19 is most distant from the limb of the wearer. It will be understood that the scope of the present invention is not limited to four layers. Also, it is understood that the configuration of the layers of the sleeve can be different than that disclosed in the illustrated embodiment.

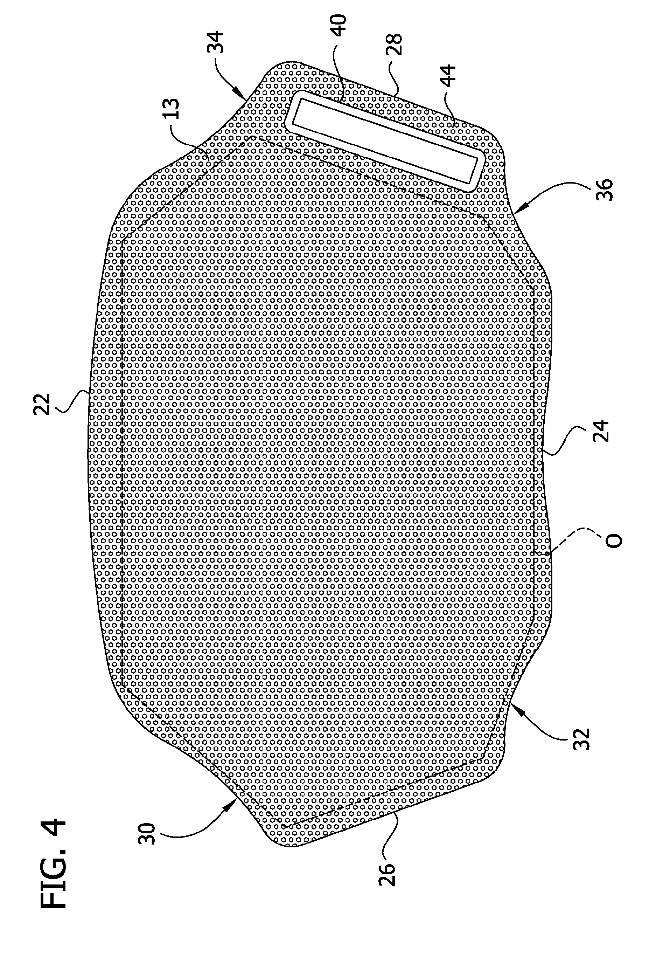

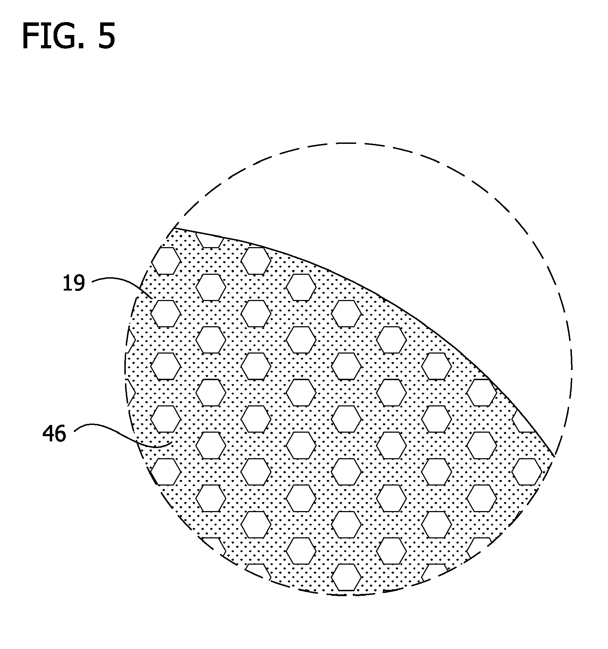

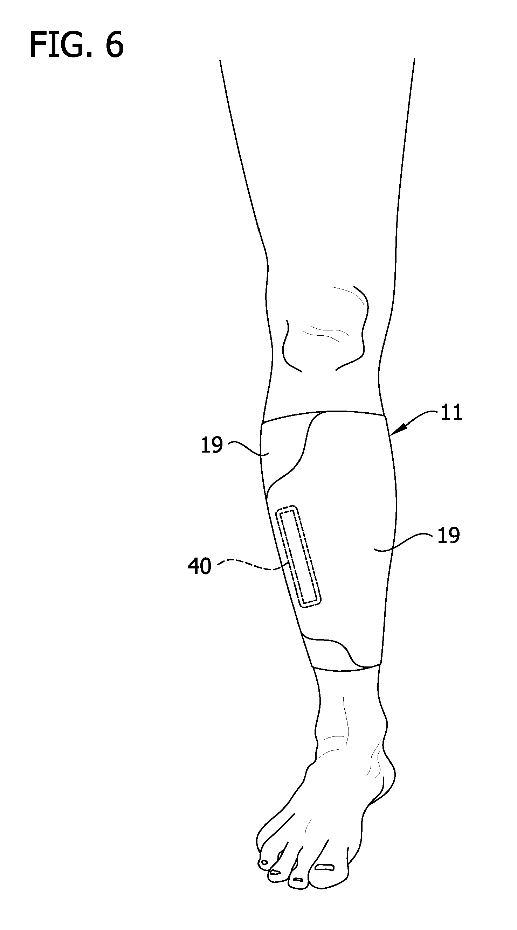

Hook component 40 (Fig. 4) is attached to an inner surface of the inner layer 13 at flap 44. An outer surface of the outer layer 19 is formed of a loop material having loops 46 (Fig. 5). This allows the hook components 40 to be secured anywhere along the outer surface of the outer cover 19 when the sleeve 11 is wrapped circumferentially around the leg of the wearer (Fig. 6). This allows for sleeve 11 to be of a substantially one-size-fits-all configuration with respect to the circumferences of different wearers' legs. Moreover, the outer cover 19 having the loops 46 allows the practitioner to quickly secure the sleeve 11 to the wearer's limb without needing to align the hook components 40. Other structure may be used as a fastener to secure the sleeve on the leg within the scope of the present invention.

The first and second intermediate layers 15, 17 respectively, each include a single sheet of air impermeable material (broadly, "bladder material"). In the illustrated embodiment, the sheets 15, 17 may be made of a pliable PVC material. The inner and outer layers 13, 19 may be made of a polyester material. The inner layer 13 can be absorbent or provide wicking, and have a soft texture to provide a comfortable interface with the wearer's body. The second intermediate layer 17 is sealingly secured to the first intermediate layer 15 along bladder seam lines 23 defining a proximal bladder 25, an intermediate bladder 27 and a distal bladder 29, respectively, that are spaced apart longitudinally along the sleeve 11. It is understood that the intermediate layers 15, 17 may be secured together at other locations, such as around their peripheries. Also, the number of bladders may be other than three without departing from the scope of the present invention. As used herein, the terms "proximal", "distal", and "intermediate" represent relative locations of components, parts and the like of the compression sleeve when the sleeve is secured to the wearer's limb. As such, a "proximal" component or the like is disposed most adjacent to a point of attachment of the wearer's limb to the wearer's torso, a "distal" component is disposed most distant from the point of attachment, and an "intermediate" component is disposed generally anywhere between the proximal and distal components.

Each bladder 25, 27, 29 may receive fluid from a source of compressed fluid (not shown) via a dedicated proximal bladder tube 31, intermediate bladder tube 33, or distal bladder tube 35, respectively for inflating the bladders. However, a tube line need not be dedicated to a bladder to practice the invention. Each tube 31, 33, 35 is disposed, at least partially, between the second intermediate layer 17 and the outer cover 19, and secured between the first and second intermediate layers 15, 17 to the respective bladders 25, 27, 29 by the respective bladder seam lines 23. Other ways of securing the tubes 31-35 to the bladders 25-29 are within the scope of the invention. Distal ends of the tubes 31, 33, 35 are ganged together using a connector 41 that is adapted to fluidly connect the tubes to the source of compressed fluid. The source of compressed fluid may be an air compressor under the control of a microprocessor that sequentially pressurizes the bladders as is generally known in the art. An exemplary air compressor is described in U.S. Patent No. 5,876,359 to Bock, the disclosure of which is incorporated herein by reference.

The upper proximal edge 22, lower distal edge 24, side edges 26, 28 and upper and lower transition edges 30, 32, 34, 36 of the sleeve 11 define a generally elongated octagonal shape of the sleeve, illustrated by the dotted outline O in Fig. 4. Edges of the sleeve may have curvature like edges 22, 24, 30, 32, 34, and 36 in the illustrated embodiment, or may be straight ("linear") like edges 26 and 28. However, the radius of curvature of each curved edge is large in comparison to the transition curvature between adjacent edges. Thus, the elongated octagon shape of the sleeve is readily appreciated. The elongated octagon shape of the sleeve 11 contours to a lower leg of a wearer better than prior sleeves while still maintaining the circumferential surface coverage of the leg exhibited by sleeves in the prior art for providing adequate compression therapy to the leg.

When inflated, the sleeves of the prior art tend to form gaps between the sleeve and the wearer's leg. These gaps inhibit the sleeve's ability to stay in place on the leg and provide the desired compression therapy to the leg. The transition edges 30, 32, 34, 36 of the sleeve 11 provide a shape that eliminates excess material that would form gaps when the sleeve 11 is wrapped around the wearer's leg and the bladders 25, 27, 29 are inflated. So the modified shape of the sleeve 11 allows the portion of the inner layer 13 which is directly opposite the wearer's leg to contact the wearer's leg without forming gaps when the sleeve 11 is wrapped around the wearer's leg and the bladders 25, 27, 29 are inflated. The sleeve 11 can provide positive location on the leg without the use of securement straps (i.e., elongate pieces of material attached to the sleeve and configured for wrapping around the wearer's body) or other means for holding the sleeve in place. Thus, the sleeve 11 relies on its shape and edge contours rather than additional strap components to keep the sleeve 11 in place on the leg resulting in more effective compression therapy to the wearer.

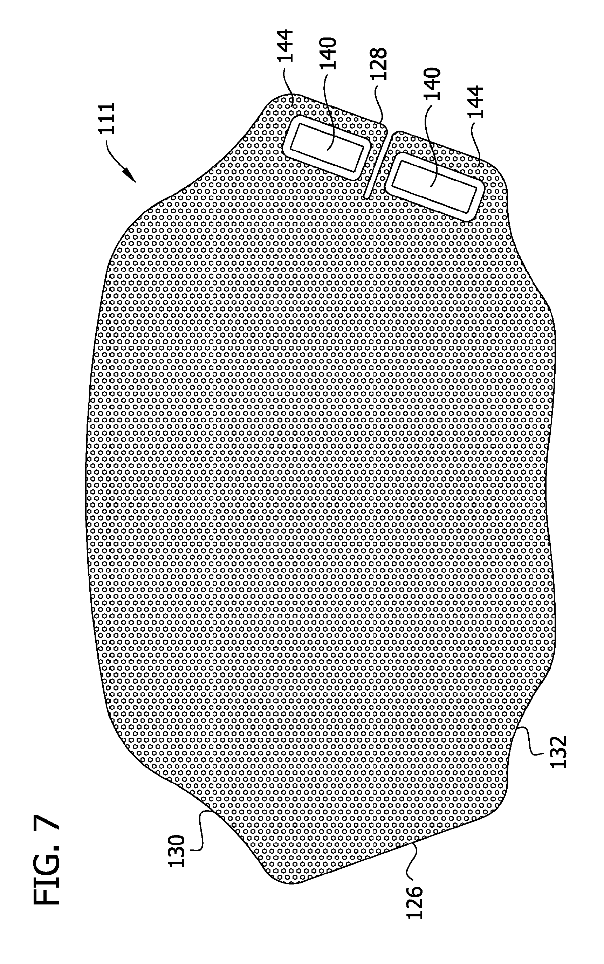

Referring to Fig. 7, a sleeve of a second embodiment of the invention is generally indicated at 111. The sleeve 111 is closely similar to the sleeve 11 of the first embodiment except the second embodiment sleeve has a pair of flaps 144 at side edge 128 each having a hook component 140.

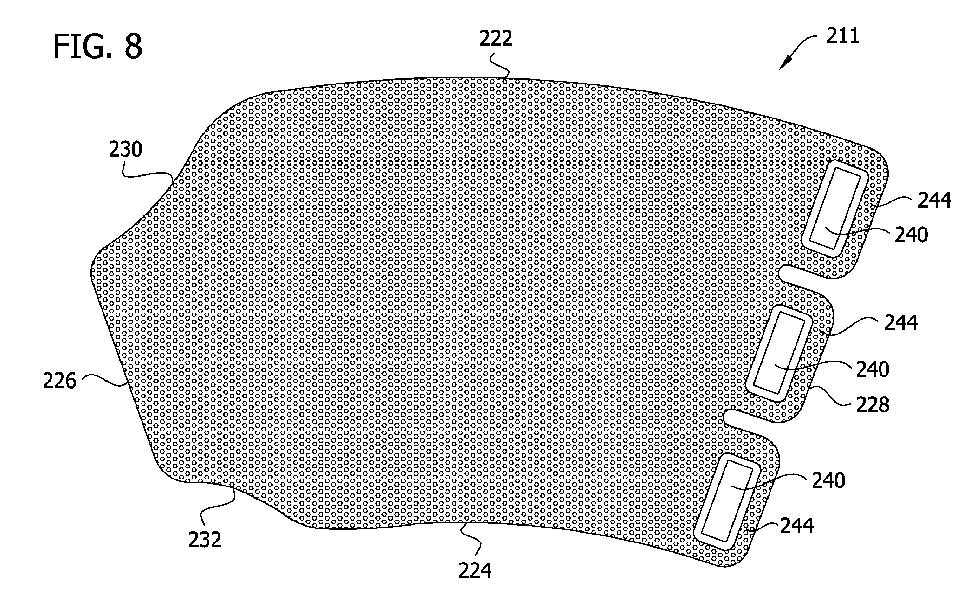

Referring to Fig. 8, a sleeve of a third embodiment of the invention is generally indicated at 211. The sleeve 211 is similar to the sleeves 11 and 111 of the first and second embodiments except the third embodiment sleeve has three flaps 244 at side edge 226 each having a hook component 240. There are no transition edges between an upper proximal edge 222 and second side edge 228, or between a lower distal edge 224 and the second side edge 228. As a result, the sleeve 211 does not have the generally elongated octagonal shape of the first and second embodiments, but has a six sided shape in the nature of an elongated, skewed hexagon. However, edges 230 and 232 provide the preferred contour for positively locating the sleeve 211 on the leg.

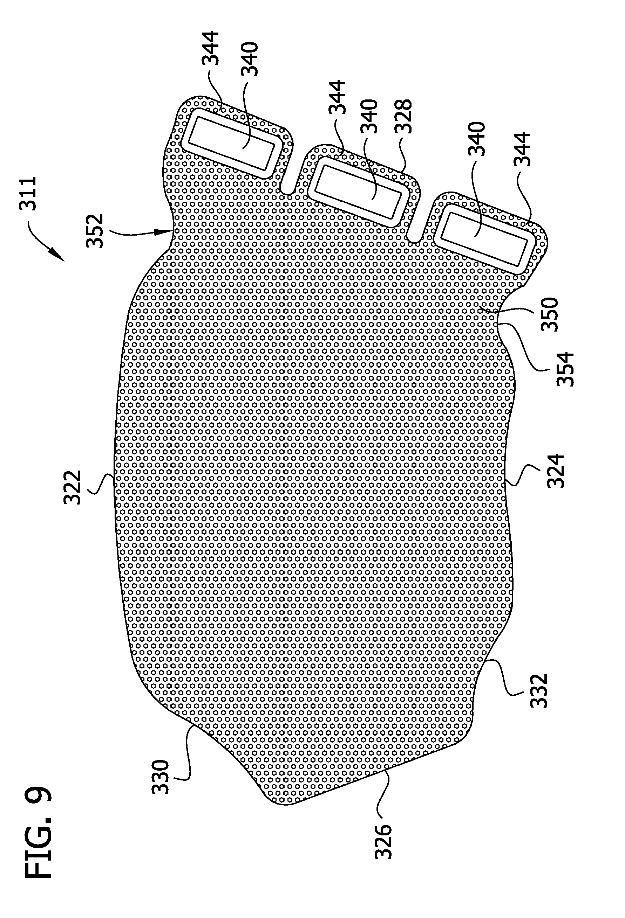

Referring to Fig. 9, a sleeve of a fourth embodiment of the invention is generally indicated at 311. The sleeve 311 is similar to the sleeve 211 of the third embodiment except the fourth embodiment sleeve includes a strap portion 350 having three flaps 344 at second side edge 328 with each flap having a hook component 340. The strap portion 350 is defined by recessed portions 352, 354. Therefore, like in the third embodiment, the sleeve 311 does not have the generally octagonal shape of the first and second embodiments. However, edges 330 and 332 extending from the upper proximal edge 322 and lower distal edge 324, respectively, provide the preferred contour for positively locating the sleeve 311 on the leg.

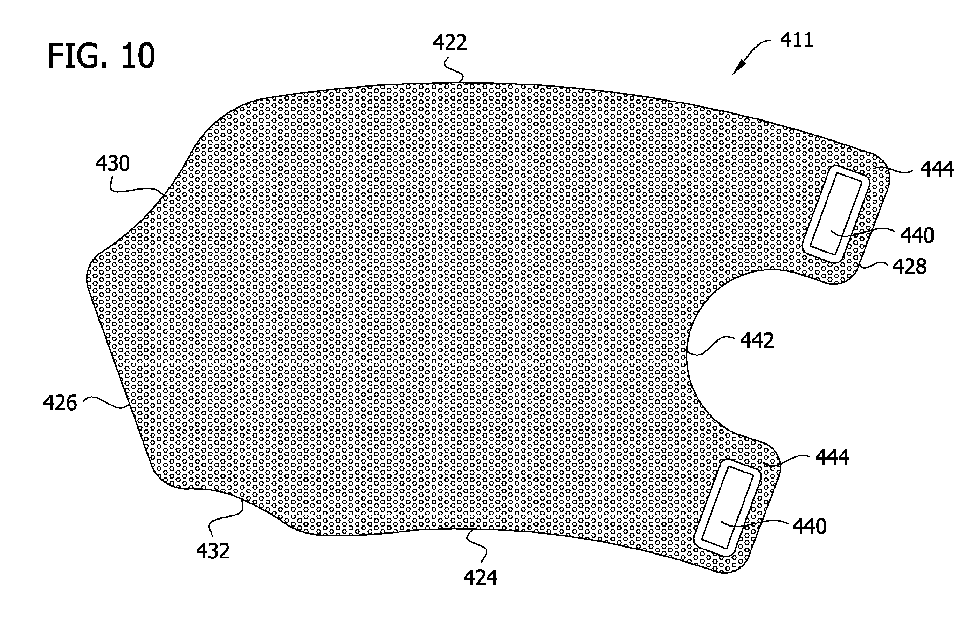

Referring to Fig. 10, a sleeve of a fifth embodiment of the invention is generally indicated at 411. The sleeve 411 is similar to the sleeve 211 of the third embodiment except the fifth embodiment only has two flaps 444 each having a hook component 440. The sleeve 411 has transition edges 430, 432 that extend between the upper proximal edge 422 and the first side edge 426, and between the lower distal edge 424 and the first side edge 426, respectively. A recess 442 extends between the flaps 444 on a second side edge 428 of the sleeve 411 to reduce the material of the sleeve and improve the overall fit by reducing the amount of material at the edge of the sleeve 411 to remove gaps that may form between the sleeve and the leg when the sleeve is worn. In general, the peripheral edges of the sleeve may be linear or curved. However when the edge is curved, it will extend through an angle about the center of curvature that is less than 180°, and most usually much less than 180°. Thus, the boundary of the recess 442 is not considered a peripheral edge of the sleeve 411.

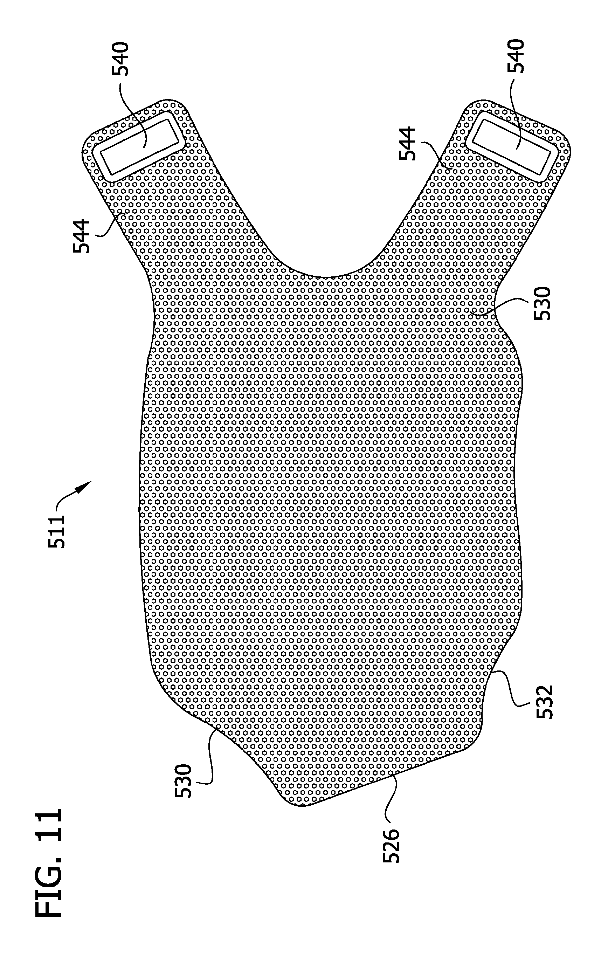

Referring to Fig. 11, a sleeve of a sixth embodiment of the invention is generally indicated at 511. The sleeve 511 is identical to the sleeve 311 of the fourth embodiment except a strap portion 530 of the sixth embodiment includes two divergent elongate flaps 544 each having a hook component 540. The flaps 544 are configured to cross each other when the sleeve 511 is wrapped around the wearer's leg.

Referring to Figs. 12 and 13, a sleeve of a seventh embodiment of the invention is generally indicated at 611. The sleeve 611 is a thigh-length sleeve having a lower leg portion 650 and a thigh portion 653. The lower leg portion 650 is closely similar to the sleeve 111 of the second embodiment. The sleeve 611 further comprises a bridge portion 655 comprising a pair of bridge sections 657 extending between the lower leg portion 650 and the thigh portion 653. The bridge sections 657 define an opening 659 disposed behind the knee of the wearer when the sleeve 611 is wrapped around the wearer's leg. The thigh portion 653 includes a main body section 661 and a pair of flaps 644 extending transversely from the main body section. The main body section 661 has a generally hexagonal shape illustrated by the dotted outline H in Fig. 12 that is configured to contour to a thigh of the wearer when the sleeve 211 is wrapped around the wearer's leg. The thigh portion 653 includes transition edges 630, 632 that each extend from an upper edge 622 to respective side edges 626, 628. The transition edges are generally linear and extend from the upper edge 622 at about a 30 degree angle α' (only one angle is shown). Alternatively, only one of the side edges 626, 628 may have a transition edge.

Having described the invention in detail, it will be apparent that modifications and variations are possible without departing from the scope of the invention defined in the appended claims.

When introducing elements of the present invention or the preferred embodiments(s) thereof, the articles "a", "an", "the" and "said" are intended to mean that there are one or more of the elements. The terms "comprising", "including" and "having" are intended to be inclusive and mean that there may be additional elements other than the listed elements.

In view of the above, it will be seen that the several objects of the invention are achieved and other advantageous results attained.

As various changes could be made in the above constructions without departing from the scope of the invention, it is intended that all matter contained in the above description and shown in the accompanying drawings shall be interpreted as illustrative and not in a limiting sense.

## Claims

1. A compression sleeve for applying compression therapy to a wearer's body, the sleeve comprising:
a panel of material including,
an upper proximal edge, a lower distal edge, first and second opposing side edges and a longitudinal axis extending from the proximal edge to the distal edge;
a first upper transition edge extending from the upper proximal edge to the first side edge, the first upper transition edge having one of a generally concave or linear shape;
a second lower transition edge extending from the lower distal edge to the first side edge, the second lower transition edge having a generally concave or linear shape;
at least one inflatable bladder attached to the panel; and
a fastener for securing the panel in a wrapped configuration around the wearer's body.

2. A compression sleeve as set forth in claim 1 wherein the first upper transition edge is greater in length than the second lower transition edge.

3. A compression sleeve as set forth in claims 1 or 2 wherein the first upper transition edge and second lower transition edge are concave in shape.

4. A compression sleeve as set forth in any one of the preceding claims wherein the panel further comprises a third upper transition edge extending from the upper proximal edge to the second side edge, the third upper transition edge having a generally concave or linear shape, and a fourth lower transition edge extending from the lower distal edge to the second side edge, the fourth lower transition edge having a generally concave or linear shape.

5. A compression sleeve as set forth in claim 4 wherein the first upper transition edge, the second lower transition edge, the third upper transition edge and the fourth lower transition edge are each concave in shape.

6. A compression sleeve as set forth in any one of the preceding claims wherein the sleeve is free of any elongate securement straps extending from the sleeve.

7. A compression sleeve as set forth in claim 1 wherein the panel has a periphery including the upper proximal edge, the lower distal edge, the first and second side edges, the first upper transition edge and second lower transition edge, the periphery having a generally elongated octagon shape.

8. A compression sleeve as set forth in claim 1 wherein the panel has a periphery including the upper proximal edge, the lower distal edge, the first and second side edges, the first upper transition edge and second lower transition edge, the periphery having a generally elongated hexagon shape.

9. A compression sleeve as set forth in any one of the preceding claims wherein the panel is split along the second side edge into flaps, and wherein there are two flaps on the second side edge each being located adjacent a respective end of the second side edge, the panel further including a recess between the two flaps having a width at least equal to the width of one of the flaps.

10. A compression sleeve as set forth in claim 9 wherein the two flaps extend from the panel in directions that diverge from one another.

11. A compression sleeve as set forth in claim 1 wherein the panel comprises a lower panel portion and an upper panel portion, the lower panel portion including the upper proximal edge, the lower distal edge, the first and second side edges, the first upper transition edge and the second lower transition edge, the upper panel portion including an upper proximal edge, a lower distal edge, first and second side edges, a first upper transition edge extending between the upper proximal edge and the first side edge, the first transition edge being generally concave or linear in shape.

12. A compression sleeve as set forth in claim 11 wherein the upper panel portion further comprises a second upper transition edge extending between the upper proximal edge and the second side edge, the second transition edge being generally concave or linear in shape.

13. A compression sleeve as set forth in claims 11 or 12 wherein the upper panel portion has a generally hexagon shape.

14. A compression garment for applying compression therapy to a limb of a wearer, the garment comprising a compression sleeve including at least one inflatable bladder, the sleeve having a periphery including at least six edges, the sleeve being configured to wrap around the wearer's limb so that the at least one bladder encircles the limb for applying compression therapy to the limb, the shape of the sleeve conforming to the limb of the wearer and keeping the sleeve in place on the limb.

15. A compression garment as set forth in claim 14 wherein the periphery of the sleeve includes eight edges arranged generally in the form of an elongated octagon.
